# EUROPEAN PATENT SPECIFICATION

(11) **EP 0 608 828 B1**
(45) Date of publication and mention of the grant of the patent: **22.03.2000**
(21) Application number: 94101051.4
(22) Date of filing: 25.01.1994
(51) Int. Cl.: A61K 31/47, A61K 47/12

(54) **Compositions containing argatroban analogs**
Arzneimittel enthaltend Argatroban Analoga
Compositions pharmaceutiques à base d'analogues d'argatroban

(30) Priority: 25.01.1993 JP 1006093
(43) Date of publication of application: 03.08.1994
(73) Proprietor: Mitsubishi Chemical Corporation, Chiyoda-ku Tokyo (JP)
(72) Inventor: Igaki, Matsuo, Kashima-gun, Ibaraki-ken (JP)
(74) Representative: Kolb, Helga, Dr. Dipl.-Chem.

(56) References cited:
- EP-A- 0 008 746
- EP-A- 0 181 267
- EP-A- 0 301 970
- DATABASE WPI Week 9026, Derwent Publications Ltd., London, GB; AN 90-196839 (26) & JP-A-02 129 123 (MITSUBISHI KASEI) 17 May 1990

## Description

The present invention relates to an antithrombin composition and a process for the preparation thereof. More particularly, this invention is directed to an antithrombin composition in the form of a lipid emulsion which comprises an arginineamide, unsaturated fatty acid and an emulsifier, and to a process for preparing the same.

Arginineamides described in Japanese Patent Application Laid-open Nos. 15267/1981, 33499/1980 and 92213/1981 are known as being useful as an antithrombin agent. Since these compounds have very low solubility in various pharmaceutically acceptable solvents, it is very difficult to prepare a highly concentrated solution thereof. For example, it is impossible to provide them in the form of a highly concentrated solution for injection.

Of known arginineamides, commercially available argatroban [(2R,4R)-1-[N²-((RS)-3-methyl-1,2,3,4-tetrahydro-8-quinolinesulfonyl)-L-arginyl]-4-methyl-2-piperidinecarboxylic acid hydrate] is a compound exhibiting selective antithrombin activity under a quite novel mechanism which has not been seen in known antithrombin agents. This compound has been administered to mammals for inhibiting the formation of thrombosis generated within the blood vessel.

Since argatroban itself is hardly soluble in various pharmaceutically acceptable conventional solvents, it is usually administered in the form of an intravenous drip injection so that 10 mg/20 ml of argatroban may be administered over 2 to 3 hours twice a day.

In such a situation as mentioned above, there has been an earnest desire to develop an administering system of antithrombin agents including argatroban, which system is applicable to all thrombin-associated conditions, except for chronic arteriosclerosis, such as postoperation of heart or blood vessel, various thrombosis diseases inclusive of cerebral thrombosis, acute cardiomuscular sclerosis, pulmonary infarction or the like, prevention of entoptic postoperative fibrin formation during the surgery of amphiblostrodes, vitreous body, cataract, glaucoma or the like diseases, inhibition of coagulation of perfusion blood during extracorporeal blood circulation and the like, where the unique actions possessed by antithrombin agents, such as the actions inhibiting ① depression of fibrin formation, ② stabilization of fibrin by activation of factor XIII, ③ platelet aggregation, due to thrombin, are highly desirable.

On the other hand, a pharmaceutical formulation in the form of a lipid emulsion has been produced in an attempt to increase pharmacological activity of various pharmacologically active substances. It is well known that the lipid emulsion has a property of moving from blood or other application site to reticulo-endothelial system, inflamatory region, or arterialsclerosis region of vascular wall, or a certain cancer, and it has been used in a targeting therapy.

For example, there have been reported a lipid emulsion comprising a vegetable oil, phosphatidyl choline, aiming at accelerating disappearing rate of Ubidecalenon from serum, which is known to be effective for improving cardiac function (Japanese Patent Application Laid-open No. 56124/1986), a lipid microsphere carrying superoxide dismutase (SOD) bound to its surface by means of a covalent bond, in order for transporting SOD only to the affected region at high concentration (Japanese Patent Application Laid-open No. 253031/1988), a lipid emulsion in which a particle diameter of emulsion drops has been minimized for inhibiting the lowering of serum concentration of a lipophilic active ingredient (Japanese Patent Application Laid-open No. 143826/1989), a lipid emulsion consisting of water-isoluble active ingredient, vegetable oil, and surfactant, in which emulsion drops have been micronized (Japanese Patent Application Laid-open No. 226807/1989), a lipid microsphere on which an acitive ingredient having antiarterosclerotic activity has been adsorbed (Japanese Patent Application Laid-open No. 294626/1989), and the like. These emulsions are reported to have provided clinically excellent results. Most of these reports are associated with lipid emulsions containing vegetable oils, typically soybean oil, and lecithin, but there is no report about a stabilized lipid emulsion which employs only an unsaturated fatty acid as an oil and fat component.

An object of the present invention was to find a solvent which is capable of improving the solubility of arginineamides inclusive of argatroban, to produce high concentrated formulations than known injections with respect to the arginineamides, and to develop enteral or parenteral formulations clinically applicable to previously-noted postoperative treatment, various thrombosis, thrombotic diseases, and the like.

As the results of intensive study, the present inventors have found that the arginineamides are very easily soluble in unsaturated fatty acid. On the basis of this finding, the inventors have succeeded in preparing a lipid emulsion containing an arginineamide, which is stable against long-term storage and sterilization by heating. This finding has also permitted the development of various types of formulation for arginineamides.

Accordingly, the present invention provides anti-thrombin composition in the form of a lipid emulsion which comprises, together with unsaturated fatty acid and an emulsifier, N²-arylsulfonyl-L-arginineamide represented by the following general formula (I): 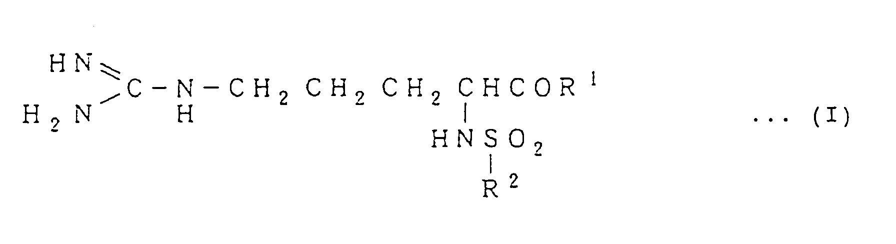 wehrein R¹ represents (2R,4R)-4-alkyl-2-carboxypiperidino group, R² represents phenyl group or condensed polycyclic compound residue as defined below, said R² being optionally substituted by one or more substituents selected from lower alkyl group, lower alkoxy group, or amino group substituted by lower alkyl group, said condensed polycyclic compound residue being a condensed polycyclic compound residue including a benzene ring, the benzene ring being bound to the sulfur atom of the sulfonyl group in the general formula (I), and said benzene ring being condensed with other ring which may be a heterocyclic ring, and said polycyclic compound residue having 7 - 14 carbon atoms in total, its hydrate and/or its salt. The present invention also provides a process for preparing the above-identified pharmaceutical composition.

The present invention will be explained in detail below.

R¹ in the general formula (I) represents (2R,4R)-4-alkyl-2-carboxypiperidino group, wherein "alkyl" means C₁ - C₅ lower alkyl group such as methyl group, ethyl group, propyl group, isopropyl group or butyl group.

R² represents phenyl group or condensed polycyclic compound residue as defined below.

The condensed polycyclic compound residue is a condensed polycyclic compound residue including benzene ring, said benzene ring being bound to the sulfur atom of the sulfonyl group in the general formula (I) and said benzene ring being condensed with other ring which may be a heterocyclic ring, and said polycyclic compound residue having 7 - 14 carbon atoms in total.

More preferably, the condensed polycyclic compound residue is a bicyclic compound residue or tricyclic compound residue. In the case of the bicyclic compound residue, the benzene ring may be preferably condensed with 5-membered ring or 6-membered ring compound, and said 5-membered ring or 6-membered ring compound may he heterocyclic compound. In the case of the tricyclic compound residue, a 5-membered ring or 6-membered ring compound may be condensed with another 5-membered ring or 6-membered ring compound, and the latter may also be heterocyclic. The heteroatom constituting such a heterocyclic compound may be oxygen atom, nitrogen atom, or sulfur atom.

Further, R² may be substituted by one or more substituents selected from lower alkyl group, lower alkoxy group, or amino group substituted by lower alkyl group. The lower alkyl group means C₁ - C₅ alkyl group such as methyl group, ethyl group, propyl group, isopropyl group, butyl group, isobutyl group, or tert-butyl group. The lower alkoxy group means C₁ - C₅ alkoxy group such as methoxy group, ethoxy group, propoxy group, isopropoxy group, or butoxy group. The amino group substituted by lower alkyl group includes alkylamino group and dialkylamino group substituted by said lower alkyl group.

Specific examples of the condensed polycyclic compound residue represented by R₂ include anthryl group, phenanthryl group, benzfuranyl group, dibenzothienyl group, phenoxazinyl group, quinolyl group, carbazolyl group, acridinyl group, phenazinyl group, phenothiazinyl group, phenoxadinyl group, benzimidazolyl group, fluorenyl group, 2,3-dihydrobenzofuranyl group, thioxanthenyl group, naphthyl group, tetrahydronaphthyl group, isoquinolyl group, tetrahydroquinolyl group, tetrahydroisoquinolyl group and the like. The benzene group of said polycyclic compound residue is combined with the sulfur atom of the sulfonyl group in the general formula (I), but the binding position on the benzene ring is not limitative.

Specific examples of the compounds of the present invention are shown below.
(2R,4R)-1-[N²-(3-isopropoxybenzenesulfonyl)-L-arginyl]-4-methyl-2-piperidinecarboxylic acid,
(2R,4R)-1-[N²-(3,5-dimethyl-4-propoxybenzenesulfonyl)-L-arginyl]-4-methyl-2-piperidinecarboxylic acid,
(2R,4R)-1-[N²-(5,6,7,8-tetrahydro-2-naphthalenesulfonyl)-L-arginyl]-4-methyl-2-piperidinecarboxylic acid,
(2R,4R)-1-[N²-(5-dimethylamino-1-naphthalenesulfonyl)-L-arginyl]-4-methyl-2-piperidinecarboxylic acid,
(2R,4R)-1-[N²-(3-methyl-1,2,3,4-tetrahydro-8-quinoline-sulfonyl)-L-arginyl]-4-methyl-2-piperidinecarboxylic acid,
(2R,4R)-1-[N²-(2-dibenzothiophenesulfonyl)-L-arginyl]-4-methyl-2-piperidinecarboxylic acid,
(2R,4R)-1-[N²-(2,4-dimethoxy-3-butoxybenzenesulfonyl)-L-arginyl]-4-methyl-2-piperidinecarboxylic acid,
(2R,4R)-1-[N²-(3,5-dimethyl-4-propoxybenzenesulfonyl)-L-arginyl]-4-methyl-2-piperidinecarboxylic acid,
(2R,4R)-1-[N²-(3-ethyl-1,2,3,4-tetrahydro-8-quinolinesulfonyl)-L-arginyl]-4-methyl-2-piperidinecarboxylic acid,
(2R,4R)-1-[N²-(2-carbazolesulfonyl)-L-arginyl]-4-methyl-2-piperidinecarboxylic acid,
(2R,4R)-1-[N²-(2-fluorenesulfonyl)-L-arginyl]-4-methyl-2-piperidinecarboxylic acid,
(2R,4R)-1-[N²-(2-phenoxazinesulfonyl)-L-arginyl]-4-methyl-2-piperidinecarboxylic acid,
(2R,4R)-1-[N²-(2-anthracenesulfonyl)-L-arginyl]-4-methyl-2-piperidinecarboxylic acid,
(2R,4R)-1-[N²-(7-methyl-2-naphthalenesulfonyl)-L-arginyl]-4-methyl-2-piperidinecarboxylic acid, and the like. Among them, previously-mentioned argatroban is most preferred.

The above compounds can exist in the form of optical isomers and diastereomers, and the arginineamides of the present invention include all of them.

The arginineamides can be easily prepared, for example, according to the method as described in Japanese Patent Application Laid-open No. 15267/1981.

The arginineamides of the present invention can be converted into acid addition salts thereof with various inorganic acids or organic acids, inorganic bases or organic bases according to the method as described in Japanese Patent Application Laid-open No. 15267/1981. Moreover, their hydrates can be prepared in a conventional manner.

The unsaturated fatty acids include oleic acid, linolic acid, linolenic acid and the like, but no limitation is given as far as they are pharmaceutically acceptable. However, oleic acid is most preferred.

The emulsifiers include soybean lecithin or hydrogenated soybean lecithin, and surfactants such as polyoxyethylene hardened castor oil, polyoxyethylene sorbitan fatty acid ester, sucrose fatty acid ester, glycerin fatty acid ester, sorbitan fatty acid ester or the like, and they can be used singly or in a combination thereof. A combination of soybean lecithin or hydrogenated soybean lecithin with a surfactant is preferred.

The antithrombin composition of the present invention includes 0.01 - 3% by weight of an arginineamide, 1 - 50% by weight of unsaturated fatty acid, and 0.1 - 10% , preferably 0.1 - 5%, by weight of an emulsifier with repsect to the total weight of the composition.

If necessary, the antithrombin composition of the present invention can include pharmaceutically acceptable isotonic agents such as sodium chloride, glycerin or the like, pH regulators such as sodium acetate, citric acid, sodium phosphate, boric acid-borax or the like, preservatives such as p-oxybenzoic acid ester, benzalkonium chloride, benzethonium chloride or the like, and antioxidants or stabilizers such as α-tocopherol or the like.

The antithrombin composition of the present invention can be prepared, for example, in the manner as shown below. Thus, an emulsifier is dissolved or dispersed in an appropriate amount of water, and other ingredients such as isotonic agents, stabilizers, preservatives or the like may be added if necessary. The resultant mixture is warmed at 70 - 80°C, mixed with an oily phase previously prepared by dissolving an arginineamide in unsaturated fatty acid, and the mixture is subjected to crude emulsification using a conventional emulsifying equipment. The resultant crude emulsion is subjected to elaborate emulsification using a high pressure emulsifying equipment over an appropriate period of time, and the resultant lipid emulsion is charged into an appropriate vessel and sterilized with high pressure steam to give the final formulation.

As emulsifying equipment usable for crude emulsification there are exemplified Desktop High-speed Homomixer manufactured by Mizuho Kogyo Company Ltd., Desktop T. K Homomixer manufactured by Tokushu Kika Kogyo Company Ltd., and the like.

As emulsifying equipment usable for elaborate emulsification there are exemplified Nanomizer manufactured by Sayama Company Ltd., Manton Golin Homonizer manufactured by Manton Golin Company Ltd., and the like.

Preferable production of the antithrombin composition of this invention is conducted in the following manner. An emulsifier, such as soybean lecithin or hydrogenated lecithin, and a surfactant are dissolved and dispersed in an appropriate amount of water, and the mixture is admixed with other ingredients such as isotonic agents, stabilizers, preservatives, or the like if necessary. The mixture is warmed at 70 - 80°C and mixed with an oil phase previously prepared by mixing an unsaturated fatty acid, an arginineamide, and a surfactant.

The antithrombin composition of the present invention thus obtained is composed of particles having a diameter of about 0.2 µm, shows excellent stability against long-term storage and heat sterilization, and can inhibit the formation of thrombosis and fibrin, accelerate the dissolution of thrombosis, and maintain and improve the topical blood circulation, without showing decomposition of the active ingredient, enlargement of the particle diameter, and separation of an oily layer.

The antithrombin composition of this invention can be administered intravenously in the form of an injection. However, it can also be administered intraarterially, intramuscularly or subcutaneously. As non-injection routes, there are also available both enteral and parenteral routes. For parenteral route, an eye drop, a nasal drop, an external drug, a suppository and the like may be used. In any case, pharmaceutically acceptable additives such as bases, excipients, or the like may be added as necessary.

The antithrombin composition of the invention shows excellent storage and heat stability, without showing decomposition of the active ingredient, enlargement of the particle diameter and separation of an oily layer. Accordingly, it is possible to prepare various types of formulation using the composition.

Thus, the antithrombin composition of the invention can be used, for example, as an injection which is administrable in a short time once a day, or as non-injectable formulations such as formulations for oral use, an eye drop, a nasal drop, an external formulation, a suppository, or the like. The formulations are used for the treatment after surgery, for various thrombotic diseases, for the treatment after entoptic surgery, for extracorporeal circulation, or the like.

### Example 1.

A mixture of soybean lecithin (1.5 g), glycerin (2.5 g), polyoxyethylene hardened castor oil (Nikkol HCO-60) (0.9 g) and sodium hydrogensulfite (0.05 g) was dissolved or dispersed in an appropriate amount of water while heating at 70 - 80°C. A solution of argatroban [(2R,4R)-1-[N²-(RS)-3-methyl-1,2,3,4-tetrahydro-8-quinolinesulfonyl)-L-arginyl]-4-methyl-2-piperidinecarboxylic acid hydrate] (0.5 g) and sucrose fatty acid ester (Mitsubishi Kasei Foods Sugar Ester S-770) (0.6 g) in oleic acid (10 g), which solution has previously been heated at 70 - 80°C, was added thereto and subjected to crude emulsification using a desktop high speed homomixer at 5000 rpm for 10 minutes. This crude emulsion was subjected to elaborate emulsification using Nanomizer to give a lipid emulsion having average particle diameter of about 0.2 µm. The emulsion was subjected to steam sterilization in an autoclave at 121°C for 20 minutes to give an antithrombin composition of the present invention.

### Example 2.

A mixture of soybean lecithin (1.5 g), glycerin (2.5 g), polyoxyethylene hardened castor oil (Nikkol HCO-60) (0.9 g) and sodium hydrogensulfite (0.05 g) was dissolved or dispersed in an appropriate amount of water while heating at 70 - 80°C. A solution of argatroban (1.0 g) and glycerin fatty acid ester (Riken Vitamin J-0381) (0.5 g) in oleic acid (20 g), which has previously been heated at 70 - 80°C, was added thereto and treated in the same manner as in Example 1 to give an antithrombin composition of the present invention.

### Example 3.

A mixture of soybean lecithin (1.2 g), glycerin (2.5 g), polyoxyethylene sorbitan fatty acid ester (Nikkol TO-10M) (0.9 g) and sodium hydrogensulfite (0.05 g) was dissolved or dispersed in an appropriate amount of water while heating at 70 - 80°C. A solution of argatroban (1.0 g) and glycerin fatty acid ester (Riken Vitamin J-0381) (0.5 g) in oleic acid (20 g), which has previously been heated at 70 - 80°C, was added thereto and treated in the same manner as in Example 1 to give an antithrombin composition of the present invention.

### Example 4.

A mixture of hydrogenerated soybean lecithin (1.5 g), glycerin (2.5 g), polyoxyethylene hardened castor oil (Nikkol HCO-60) (0.9 g) and sodium hydrogensulfite (0.05 g) was dissolved or dispersed in an appropriate amount of water while heating at 70 - 80°C. A solution of argatroban (1.0 g) and sucrose fatty acid ester (Mitsubishi Kasei Foods Sugar Ester S-770) (0.6 g) in oleic acid (20 g), which has previously been heated at 70 - 80°C, was added thereto and treated in the same manner as in Example 1 to give an antithrombin composition of the present invention.

### Example 5.

A mixture of hydrogenated soybean lecithin (1.2 g), glycerin (2.5 g), polyoxyethylene sorbitan fatty acid ester (Nikkol TO-10M) (0.6 g) and sodium hydrogensulfite (0.05 g) was dissolved or dispersed in an appropriate amount of water while heating at 70 - 80°C. A solution of argatroban (1.0 g) and glycerin fatty acid ester (Riken Vitamin J-0381) (0.4 g) in oleic acid (10 g), which has previously been heated at 70 - 80°C, was added thereto and treated in the same manner as in Example 1 to give an antithrombin composition of the present invention.

### Example 6.

A mixture of soybean lecithin (1.2 g), glycerin (2.5 g), polyoxyethylene hardened castor oil (Nikkol HCO-60) (0.6 g) and sodium hydrogensulfite (0.05 g) was dissolved or dispersed in an appropriate amount of water while heating at 70 - 80°C. A solution of argatroban (1.0 g) and sorbitan fatty acid ester (Nikkol SO-15R) (0.4 g) in oleic acid (10 g), which has previously been heated at 70 - 80°C was added thereto and treated in the same manner as in Example 1 to give an antithrombin composition of the present invention.

### Example 7.

A mixture of soybean lecithin (1.5 g), glycerin (2.5 g), polyoxyethylene hardened castor oil (Nikkol HCO-60) (0.9 g) and sodium hydrogensulfite (0.05 g) was dissolved or dispersed in an appropriate amount of water while heating at 70 - 80°C. A solution of argatroban (0.5 g) and polyoxyethylene hardened castor oil (Nikkol HCO-10) (0.9 g) in oleic acid (10 g), which has previously been heated at 70 - 80°C, was added thereto and treated in the same manner as in Example 1 to give an antithrombin composition of the present invention.

### Example 8.

A mixture of soybean lecithin (1.5 g), glycerin (2.5 g), polyoxyethylene hardened castor oil (Nikkol HCO-60) (0.9 g) and sodium hydrogensulfite (0.05 g) was dissolved or dispersed in an appropriate amount of water while heating at 70 - 80°C. A solution of argatroban (0.5 g) and glycerin fatty acid ester (Riken Vitamin K-37) (0.6 g) in oleic acid (10 g), which has previously been heated at 70 - 80°C, was added thereto and treated in the same manner as in Example 1 to give an antithrombin composition of the present invention.

### Example 9.

A mixture of soybean lecithin (1.5 g), glycerin (2.5 g), polyoxyethylene hardened castor oil (Nikkol HCO-60) (0.9 g) and sodium hydrogensulfite (0.05 g) was dissolved or dispersed in an appropriate amount of water while heating at 70 - 80°C. A solution of argatroban (0.5 g) and sucrose fatty acid ester (Mitsubishi Kasei Foods Sugar Ester S-970) (0.6 g) in oleic acid (15 g), which has previously been heated at 70 - 80°C, was added thereto and treated in the same manner as in Example 1 to give an antithrombin composition of the present invention.

### Experiment 1.

The antithrombin compositions obtained in Examples 1, 2 and 4 were subjected to the measurement of particle size using Sub-micron Particle Analyzer (Coulter N4, manufactured by Nikkaki Company Ltd.) before and after high pressure steam sterilization (121°C, 20 minutes).

Table 1 shows the result.

**Table 1**

| Example | Before Sterilization (nm) | After Sterilization (nm) |
|---|---|---|
| 1 | 228 | 234 |
| 2 | 214 | 218 |
| 4 | 276 | 268 |

### Experiment 2.

The antithrombin compositions obtained in Examples 1, 2 and 4 were stored in an incubator at 5°C for one month, and possible crystallization of argatroban and change of particle size of the lipid emulsion were observed.

Table 2 shows the result.

**Table 2**

| Example | Crystallization | Change of Particle Size |
|---|---|---|
| 1 | None | Unchanged |
| 2 | None | Unchanged |
| 4 | None | Unchanged |

### Experiment 3.

The antithrombin compositions obtained in Examples 1, 2 and 4 were stored in an incubator at different temperatures indicated in Table 3 for one month, and the stability of the composition was examined.

Table 3 shows the result.

**Table 3**

| Example | | 1 | 2 | 4 |
|---|---|---|---|---|
| Room Temperature | Particle Size | Unchanged | Unchanged | Unchanged |
| | Separation of Oily Layer | None | None | None |
| 5°C * | Particle Size | Unchanged | Unchanged | Unchanged |
| ↑ | | | | |
| ↓ | | | | |
| 40°C | Separation of Oily Layer | None | None | None |
| 40°C | Particle Size | Unchanged | Unchanged | Unchanged |
| | Separation of Oily Layer | None | None | None |

| | | | | |
|---|---|---|---|---|
| * Storage temperature was changed from 5°C to 40°C and vice versa repeatedly. | | | | |

The results of the above experiments revealed that the antithrombin composition of the present invention have excellent storage stability while showing neither crystallization of argatroban at low temperature nor change of the particle size after sterilization.

### Comparative Example 1.

A mixture of soybean lecithin (1.5 g), glycerin (2.5 g), polyoxyethylene hardened castor oil (Nikkol HCO-60) (0.9 g), and sodium hydrogensulfite (0.05 g) was dissolved or dispersed in an appropriate amount of water while heating at 70 - 80°C. A solution of argatroban (1.0 g) and sucrose fatty acid ester (Mitsubishi Kasei Foods Sugar Ester S-770) (0.6 g) in soybean oil (20 g), which has previously been heated at 70 - 80°C, was added thereto and treated in the same manner as in Example 1. However, satisfactory lipid emulsion was not obtained because argatroban did not dissolve in the medium used.

### Comparative Example 2.

A mixture of soybean lecithin (1.5 g), glycerin (2.5 g), polyoxyethylene hardened castor oil (Nikkol HCO-60) (0.9 g) and sodium hydrogensulfite (0.05 g) was dissolved or dispersed in an appropriate amount of water while heating at 70 - 80°C. A solution of argatroban (1.0 g) and sucrose fatty acid ester (Mitsubishi Kasei Foods Sugar Ester S-770) (0.6 g) in olive oil (20 g), which has previously been heated at 70 - 80°C, was added thereto and treated in the same manner as in Example 1. However, satisfactory lipid emulsion was not obtained because argatroban did not dissolve in the medium used.

### Comparative Example 3.

A mixture of soybean lecithin (1.5 g), glycerin (2.5 g), polyoxyethylene hardened castor oil (Nikkol HCO-60) (0.9 g) and sodium hydrogensulfite (0.05 g) was dissolved or dispersed in an appropriate amount of water while heating at 70 - 80°C. A solution of argatroban (1.0 g) and sucrose fatty acid ester (Mitsubishi Kasei Foods Sugar Ester S-770) (0.6 g) dissolved in middle chain fatty acid, triglyceride (Nippon Oil & Fat Panasate 810) (20 g), which has previously been heated at 70 - 80°C, was added thereto and treated in the same manner as in Example 1. However, satisfactory lipid emulsion was not obtained because argatroban did not dissolve in the medium used.

## Claims

1. An antithrombin composition in the form of a lipid emulsion which comprises, together with unsaturated fatty acid and an emulsifier, N²-arylsulfonyl-L-arginineamide represented by the following general formula (I): 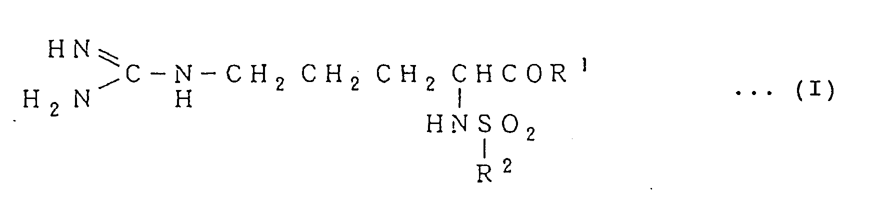 wherein R¹ represents (2R,4R)-4-alkyl-2-carboxypiperidino group, R² represents phenyl group or condensed polycyclic compound residue as defined below, said R² optionally having one or more substituents selected from C₁-C₅ alkyl group, C₁-C₅ alkoxy group , or amino group substituted by C₁-C₅ alkyl group, said condensed polycyclic compound residue being a condensed polycyclic compound residue including a benzene ring, said benzene ring being bound to the sulfur atom of the sulfonyl group in the general formula (I), and said benzene ring being condensed with other ring which may be a heterocyclic ring, and said polycyclic compound residue having 7 - 14 carbon atoms in total, its hydrate and/or its salt.

2. An antithrombin composition according to claim 1, in which R¹ is (2R,4R)-4-methyl-2-carboxypiperidino group and R² is 3-methyl-1,2,3,4-tetrahydro-8-quinolyl group.

3. All antithrombin composition according to claim 1 or 2, in which said unsaturated fatty acid is oleic acid.

4. An antithrombin composition according to claim 1 or 2, in which said emulsifier is a combination of soybean lecithin or hydrogenerated soybean lecithin and a surfactant.

5. An antithrombin composition according to claim 4, in which said surfactant is one or more components selected from polyoxyethylene hardened castor oil, sucrose fatty acid ester, sorbitan fatty acid ester, glycerin fatty acid ester, and polyoxyethylene sorbitan fatty acid ester.

6. An antithrombin composition according to any one of claims 1 - 5, which is used for inhibiting production of thrombosis and fibrin, accelerating dissolution of thrombosis, or maintaining and improving topical blood circulation.

7. An antithrombin composition according to any one of claims 1 - 6, which is in the form of an enteral or parenteral formulation.

8. A process for preparing the antithrombin composition according to claim 1, which comprises mixing and emulsifying N²-arylsulfonyl-L-arginineamide of the following general formula (I): 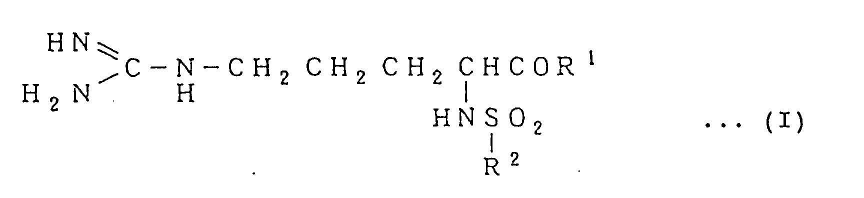 wherein R¹ represents (2R,4R)-4-alkyl-2-carboxypiperidino group, R² represents phenyl group or condensed polycyclic compound residue as defined below, said R² optionally having one or more substituents selected from C₁-C₅ alkyl group, C₁-C₅ alkoxy group, or amino group substituted by C₁-C₅ alkyl group, said condensed polycyclic compound residue being a condensed polycyclic compound residue including a benzene ring, said benzene ring being bound to the sulfur atom of the sulfonyl group in the general formula (I), and said benzene ring being condensed with other ring which may be a heterocyclic ring, and said polycyclic compound residue having 7 - 14 carbon atoms in total, its hydrate and/or its salt, unsaturated fatty acid and an emulsifier to give a lipid emulsion.

9. A process for preparing the antithrombin compositions according to claim 8, which comprises dissolving N²-arylsulfonyl-L-arginineamide, its hydrate and/or its salt, in unsaturated fatty acid, and emulsifying the resultant solution with an emulsifier to give a lipid emulsion.

10. Use of unsaturated fatty acid for improving storage stability of an emulsion containing N²-arylsulfonyl-L-arginineamide which is represented by the following general formula (I) 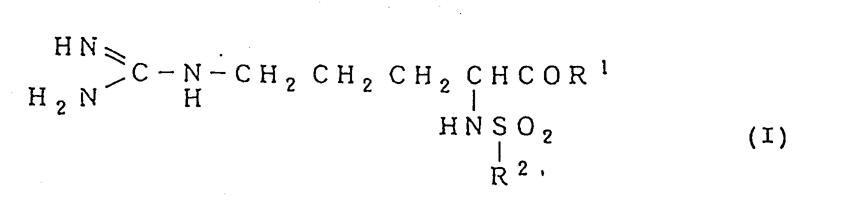 wherein R¹ represents (2R, 4R)-4-alkyl-2-carboxypiperidino group, R² represents phenyl group or condensed polycyclic compound residue as defined below, said R² optionally having one or more substituents selected from C1-C5 alkyl group, C1-C5 alkoxy group, or amino group substituted by C1-C5 alkyl group, said condensed polycyclic compound residue being a condensed polycyclic compound residue including a benzene ring, said benzene ring being bound to the sulfur atom of the sulfonyl group in the general formula (I) and said benzene ring being condensed with other ring which may be a heterocyclic ring, and said polycyclic compound residue having 7 - 14 carbon atoms in total.

11. Use of unsaturated fatty acid as a solvent for N²-arylsulfonyl-L-arginineamide which is represented by the following general formula (I) 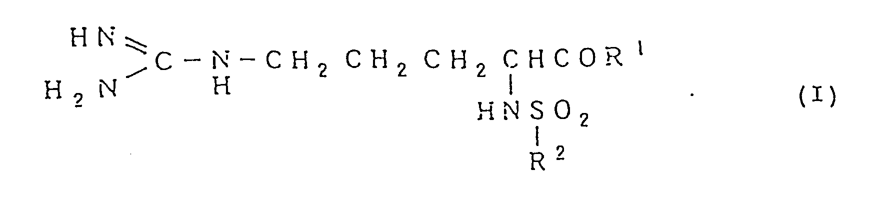 wherein R¹ represents (2R, 4R)-4-alkyl-2-carboxypiperidino group, R² represents phenyl group or condensed polycyclic compound residue as defined below, said R² optionally having one or more substituents selected from C1-C5 alkyl group, C1-C5 alkoxy group, or amino group substituted by C1-C5 alkyl group, said condensed polycyclic compound residue being a condensed polycyclic compound residue including a benzene ring, said benzene ring being bound to the sulfur atom of the sulfonyl group in said general formula (I), and said bezene ring being condensed with other ring which may be a heterocyclic ring, and said polycyclic compound residue having 7 - 14 carbon atoms in total, its hydrate and/or its salt

## Patentansprüche

1. Antithrombinzusammensetzung in Form einer Lipidemulsion, die, zusammen mit einer ungesättigten Fettsäure und einem Emulgator, N²-Arylsulfonyl-L-argininamid, dargestellt durch die folgende allgemeine Formel (I): 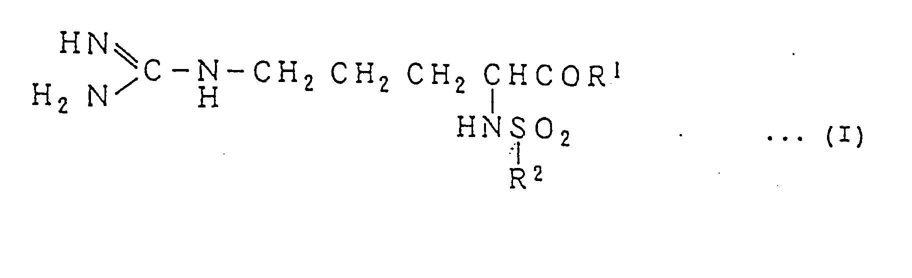 worin R¹ eine (2R,4R)-4-Alkyl-2-carboxylpiperidino-Gruppe darstellt, R² eine Phenylgruppe oder den nachstehend definierten Rest einer kondensierten polycyclischen Verbindung bedeutet und R² gegebenenfalls einen oder mehrere Substituenten aufweist ausgewählt aus einer C₁-C₅-Alkylgruppe, einer C₁-C₅-Alkoxygruppe oder einer durch eine C₁-C₅-Alkylgruppe substituierte Aminogruppe, wobei der Rest der kondensierten polcyclischen Verbindung ein Rest einer kondensierten polycyclischen Verbindung ist, die einen Benzolring umfaßt, wobei der Benzolring an das Schwefelatom der Sulfonylgruppe in der allgemeinen Formel (I) gebunden ist, und der Benzolring mit einem anderen Ring kondensiert ist, der ein heterocyclischer Ring sein kann, und der Rest der polycyclischen Verbindung insgesamt 7 bis 14 Kohlenstoffatome aufweist, sein Hydrat und/oder sein Salz, umfaßt.

2. Antithrombinzusammensetzung nach Anspruch 1, dadurch gekennzeichnet, daß R¹ eine (2R,4R)-4-Methyl-2-carboxypiperidino-Gruppe ist und R² eine 3-Methyl-1,2,3,4-tetrahydro-8-chinolylgruppe ist.

3. Antithrombinzusammensetzung nach Anspruch 1 oder 2, dadurch gekennzeichnet, daß die ungesättigte Fettsäure Ölsäure ist.

4. Antithrombinzusammensetzung nach Anspruch 1 oder 2, dadurch gekennzeichnet, daß der Emulgator eine Kombination aus Sojabohnenlecithin oder hydriertem Sojabohnenlecithin und einem oberflächenaktiven Mittel ist.

5. Antithrombinzuammensetzung nach Anspruch 4, dadurch gekennzeichnet, daß das oberflächenaktive Mittel eine oder mehrere Komponenten bedeutet, die ausgewählt sind aus Polyoxyethylen-gehärtetem Castoröl, Sucrosefettsäureester, Sorbitanfettsäureester, Glycerinfettsäureester und Polyoxyethylensorbitanfettsäureester.

6. Antithrombinzusammensetzung nach einem der Ansprüche 1 bis 5, die zur Hemmung der Bildung von Thrombose und Fibrin, zur Beschleunigung der Auflösung von Thrombose, oder zur Aufrechterhaltung und Verbesserung der topischen Blutzirkulation verwendet wird.

7. Antithrombinzusammensetzung nach einem der Ansprüche 1 bis 6, dadurch gekennzeichnet, daß sie in Form einer enteralen oder parenteralen Formulierung vorliegt.

8. Verfahren zur Herstellung der Antithrombinzusammensetzung nach Anspruch 1, das umfaßt das Mischen und Emulgieren eines N²-Arylsulfonyl-L-argininamids der folgenden allgemeinen Formel (I): 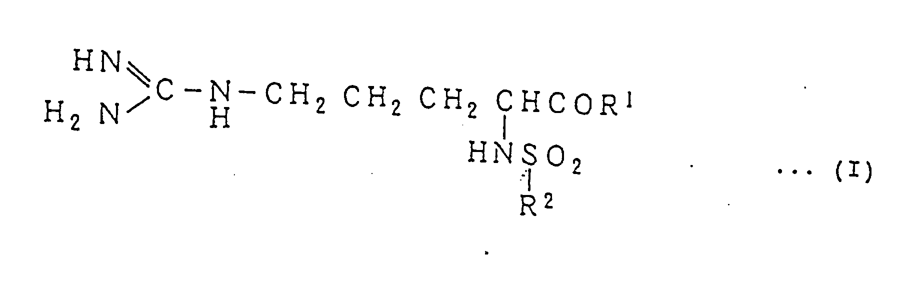 worin R¹ eine (2R,4R)-4-Alkyl-2-carboxylpiperidino-Gruppe darstellt, R² eine Phenylgruppe oder den nachstehend definierten Rest einer kondensierten polycyclischen Verbindung bedeutet und R² gegebenenfalls einen oder mehrere Substituenten aufweist ausgewählt aus einer C₁-C₅-Alkylgruppe, einer C₁-C₅-Alkoxygruppe oder einer durch eine C₁-C₅-Alkylgruppe substituierte Aminogruppe, wobei der Rest der kondensierten polcyclischen Verbindung ein Rest einer kondensierten polycyclischen Verbindung ist, die einen Benzolring umfaßt, wobei der Benzolring an das Schwefelatom der Sulfonylgruppe in der allgemeinen Formel (I) gebunden ist, und der Benzolring mit einem anderen Ring kondensiert ist, der ein heterocyclischer Ring sein kann, und der Rest der polycyclischen Verbindung insgesamt 7 bis 14 Kohlenstoffatome aufweist, seines Hydrates und/oder seines Salzes, einer ungesättigten Fettsäure und eines Emulgators unter Erhalt einer Lipidemulsion.

9. Verfahren zur Herstellung der Antithrombinzusammensetzung nach Anspruch 8, dadurch gekennzeichnet, daß es umfaßt das Auflösen eines N²-Arylsulfonyl-L-argininamids, seines Hydrates und/oder seines Salzes in einer ungesättigten Fettsäure, und Emulgieren der resultierenden Lösung mit einem Emulgator unter Erhalt einer Lipidemulsion.

10. Verwendung einer ungesättigten Fettsäure zur Verbesserung der Lagerstabilität einer Emulsion, die ein N²-Arylsulfonyl-L-argininamid enthält, das durch die folgende allgemeine Formel (I) dargestellt ist: 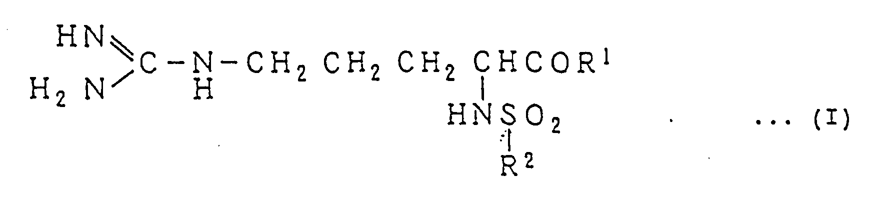 worin R¹ eine (2R,4R)-4-Alkyl-2-carboxylpiperidino-Gruppe darstellt, R² eine Phenylgruppe oder den nachstehend definierten Rest einer kondensierten polycyclischen Verbindung bedeutet und R² gegebenenfalls einen oder mehrere Substituenten aufweist ausgewählt aus einer C₁-C₅-Alkylgruppe, einer C₁-C₅-Alkoxygruppe oder einer durch eine C₁-C₅-Alkylgruppe substituierte Aminogruppe, wobei der Rest der kondensierten polcyclischen Verbindung ein Rest einer kondensierten polycyclischen Verbindung ist, die einen Benzolring umfaßt, wobei der Benzolring an das Schwefelatom der Sulfonylgruppe in der allgemeinen Formel (I) gebunden ist, und der Benzolring mit einem anderen Ring kondensiert ist, der ein heterocyclischer Ring sein kann, und der Rest der polycyclischen Verbindung insgesamt 7 bis 14 Kohlenstoffatome aufweist.

11. Verwendung einer ungesättigten Fettsäure als Lösungsmittel für N₂-Arylsulfonyl-L-argininamid, das durch die folgende allgemeine Formel (I) dargestellt wird: 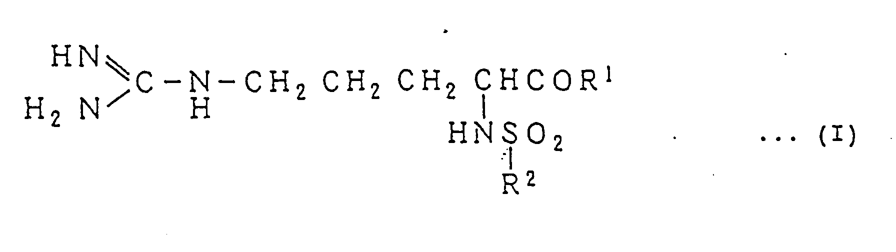 worin R¹ eine (2R,4R)-4-Alkyl-2-carboxylpiperidino-Gruppe darstellt, R² eine Phenylgruppe oder den nachstehend definierten Rest einer kondensierten polycyclischen Verbindung bedeutet und R² gegebenenfalls einen oder mehrere Substituenten aufweist ausgewählt aus einer C₁-C₅-Alkylgruppe, einer C₁-C₅-Alkoxygruppe oder einer durch eine C₁-C₅-Alkylgruppe substituierte Aminogruppe, wobei der Rest der kondensierten polcyclischen Verbindung ein Rest einer kondensierten polycyclischen Verbindung ist, die einen Benzolring umfaßt, wobei der Benzolring an das Schwefelatom der Sulfonylgruppe in der allgemeinen Formel (I) gebunden ist, und der Benzolring mit einem anderen Ring kondensiert ist, der ein heterocyclischer Ring sein kann, und der Rest der polycyclischen Verbindung insgesamt 7 bis 14 Kohlenstoffatome aufweist, sein Hydrat und/oder sein Salz.

## Revendications

1. Composition antithrombine sous la forme d'une émulsion lipidique, qui comprend, en association avec un acide gras insaturé et un émulsifiant, un N²-arylsulfonyl-L-arginine amide représenté par la formule générale (I) suivante : 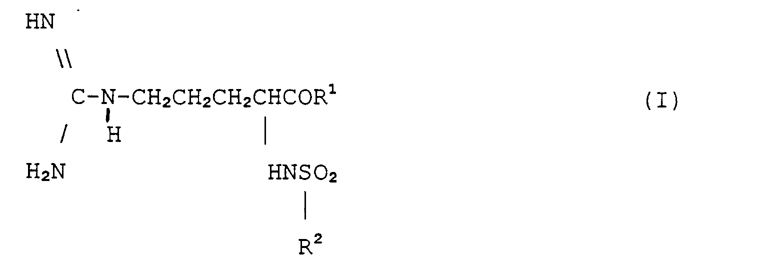 dans laquelle R¹ représente un groupe (2R,4R)-4-alkyl-2-carboxypipéridino, R² représente un groupe phényle ou un résidu de composé polycyclique condensé défini ci-dessous, ledit groupe R² comportant éventuellement un ou plusieurs substituants choisis parmi un groupe alkyle en C₁-C₅, un groupe alkoxy en C₁-C₅, ou un groupe amino substitué par un groupe alkyle en C₁-C₅, ledit résidu de composé polycyclique condensé étant un résidu de composé polycyclique condensé comprenant un cycle benzénique, ledit cycle benzénique étant lié à l'atome de soufre du groupe sulfonyle dans la formule générale (I), et ledit cycle benzénique étant condensé avec un autre noyau qui peut être un noyau hétérocyclique, et ledit résidu de composé polycyclique comportant au total de 7 à 14 atomes de carbone, un hydrate et/ou un sel de celui-ci.

2. Composition antithrombine selon la revendication 1, dans laquelle R¹ représente un groupe (2R,4R)-4-méthyl-2-carboxypipéridino, et R² représente un groupe 3-méthyl-1,2,3,4-tétrahydro-8-quinolyle.

3. Composition antithrombine selon la revendication 1 ou 2, dans laquelle ledit acide gras insaturé est l'acide oléique.

4. Composition antithrombine selon la revendication 1 ou 2, dans laquelle ledit émulsifiant est une association de lécithine de soja ou de lécithine de soja hydrogénée et d'un tensioactif.

5. Composition antithrombine selon la revendication 4, dans laquelle ledit tensioactif comprend au moins un composant choisi parmi la polyoxyéthylène-huile de ricin hydrogénée, un saccharose ester d'acide gras, un sorbitanne ester d'acide gras, un glycérine ester d'acide gras et un polyoxyéthylène sorbitanne ester d'acide gras.

6. Composition antithrombine selon l'une quelconque des revendications 1 à 5, qui est employée pour empêcher la production d'un thrombus et de la fibrine, pour accélérer la dissolution d'un thrombus, ou maintenir et améliorer la circulation sanguine topique.

7. Composition antithrombine selon l'une quelconque des revendications 1 à 6, qui est sous la forme d'un produit pour voie entérale ou parentérale.

8. Procédé de préparation de la composition antithrombine selon la revendication 1, selon lequel on mélange et on émulsifie un N²-arylsulfonyl-L-arginine amide correspondant à la formule générale (I) suivante : 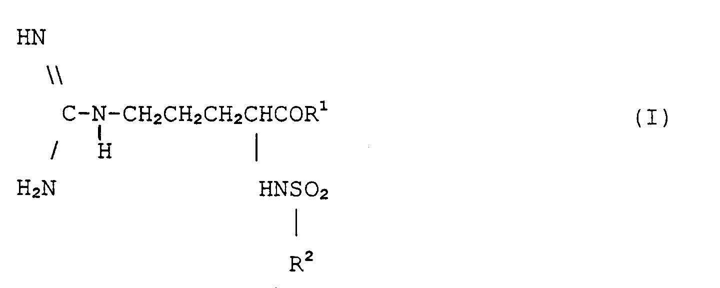 dans laquelle R¹ représente un groupe (2R,4R)-4-alkyl-2-carboxypipéridino, R² représente un groupe phényle ou un résidu de composé polycyclique condensé tel que défini ci-dessous, ledit groupe R² comportant éventuellement un ou plusieurs substituants choisis parmi un groupe alkyle en C₁-C₅, un groupe alkoxy en C₁-C₅, ou un groupe amino substitué par un groupe alkyle en C₁-C₅, ledit résidu de composé polycyclique condensé étant un résidu de composé polycyclique condensé comprenant un cycle benzénique, ledit cycle benzénique étant lié à l'atome de soufre du groupe sulfonyle dans la formule générale (I), et ledit cycle benzénique étant condensé avec un autre noyau qui peut être un noyau hétérocyclique, et ledit résidu de composé polycyclique comportant de 7 à 14 atomes de carbone au total, un hydrate et/ou un sel de celui-ci, un acide gras insaturé et un émulsifiant pour obtenir une émulsion dans un lipide.

9. Procédé de préparation des compositions antithrombine de la revendication 8, selon lequel on dissout un N²-arylsulfonyl-L-arginine amide, un hydrate et/ou un sel de celui-ci, dans un acide gras insaturé, et on émulsifie la solution résultante avec un émulsifiant afin d'obtenir une émulsion lipidique.

10. Utilisation d'un acide gras insaturé pour améliorer la stabilité à la conservation d'une émulsion contenant un N²-arylsulfonyl-L-arginine amide, représenté par la formule générale (I) suivante : 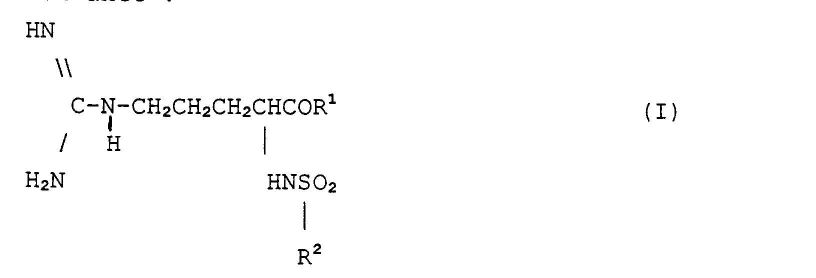 dans laquelle R¹ représente un groupe (2R,4R)-4-alkyl-2-carboxypipéridino, R² représente un groupe phényle ou un résidu de composé polycyclique condensé tel que défini ci-dessous, ledit groupe R² comportant éventuellement un ou plusieurs substituants choisis parmi un groupe alkyle en C₁-C₅, un groupe alkoxy en C₁-C₅, ou un groupe amino substitué par un groupe alkyle en C₁-C₅, ledit résidu de composé polycyclique condensé étant un résidu de composé polycyclique condensé comprenant un cycle benzénique, ledit cycle benzénique étant lié à l'atome de soufre du groupe sulfonyle dans la formule générale (I), et ledit cycle benzénique étant condensé à un autre noyau qui peut être un noyau hétérocyclique, et ledit résidu de composé polycyclique comportant au total de 7 à 14 atomes de carbone.

11. Utilisation d'un acide gras insaturé comme solvant d'un N²-arylsulfonyl-L-arginine amide, représenté par la formule générale (I) suivante : 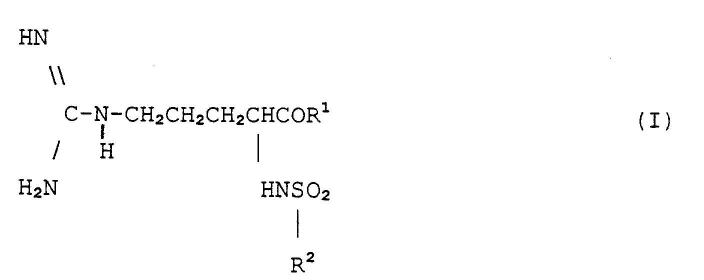 dans laquelle R¹ représente un groupe (2R,4R)-4-alkyl-2-carboxypipéridino, R² représente un groupe phényle ou un résidu de composé polycyclique condensé tel que défini ci-dessous, ledit groupe R² comportant éventuellement un ou plusieurs substituants choisis parmi un groupe alkyle en C₁-C₅, un groupe alkoxy en C₁-C₅, ou un groupe amino substitué par un groupe alkyle en C₁-C₅, ledit résidu de composé polycyclique condensé étant un résidu de composé polycyclique condensé comprenant un noyau benzénique, ledit noyau benzénique étant lié à l'atome de soufre du groupe sulfonyle dans ladite formule générale (I), et ledit cycle benzénique étant condensé avec un autre noyau qui peut être un noyau hétérocyclique, et ledit résidu de composé polycyclique comportant au total de 7 à 14 atomes de carbone, un hydrate et/ou un sel de celui-ci.
